# EUROPÄISCHE PATENTSCHRIFT

(11) **EP 1 335 710 B1**
(45) Veröffentlichungstag und Bekanntmachung des Hinweises auf die Patenterteilung: **27.12.2006**
(21) Anmeldenummer: 01993459.5
(22) Anmeldetag: 08.11.2001
(51) Int. Cl.: A61K 9/48

(54) **WEICHKAPSELN UMFASSEND EIN STÄRKEGEMISCH VERRINGERTEN VERZWEIGUNGSGRADES**
SOFT CAPSULES COMPRISING A STARCH MIXTURE HAVING A REDUCED BRANCHING DEGREE
CAPSULES SOUPLES COMPRENANT UN MELANGE D'AMIDON A DEGRE DE RAMIFICATION REDUIT

(30) Priorität: 09.11.2000 DE 10055526; 02.04.2001 CH 614012001
(43) Veröffentlichungstag der Anmeldung: 20.08.2003
(73) Patentinhaber: Celanese Ventures GmbH, 65926 Frankfurt am Main (DE)
(72) Erfinder: HAUSMANNS, Stephan, 65185 Wiesbaden (DE); KIY, Thomas, 65929 Frankfurt am Main (DE); TOMKA, Ivan, CH-8057 Zürich (CH); MÜLLER, Rolf, CH-8055 Zürich (CH)
(74) Vertreter: Dörr, Klaus
(86) Internationale Anmeldenummer: PCT/EP2001/012935
(87) Internationale Veröffentlichungsnummer: WO 2002/038132

(56) Entgegenhaltungen:
- WO-A-92/09274
- WO-A-99/02600
- WO-A1-01/85836
- DE-A- 10 022 095
- DE-A- 19 852 826
- US-A- 4 306 059

## Beschreibung

Die vorliegende Erfindung betrifft ein Verfahren zur Herstellung von proteinfreien Weichkapseln, umfassend ein Gel als Weichkapselhülle aus einem Stärkegemisch und einem Quellmittel, wobei das Stärkegemisch mindestens eine Stärkekomponente umfasst, die gegenüber nativer Stärke einen verringerten Verzweigungsgrad aufweist, und wobei das Stärkegemisch zusätzlich auch native Stärke aufweisen kann.

Die Verwendung von Weichkapseln zur Verkapselung pharmakologisch, veterinärmedizinisch, kosmetisch oder agrochemisch wirksamen Substanzen ist seit Jahren hinlänglich bekannt. Dabei kommen in der Regel Weichkapseln zur Anwendung, die neben Weichmachern und anderen gängigen Inhaltsstoffen bevorzugt aus Gelatine bestehen. Gelatine ist ein Polypeptid, das vornehmlich durch Hydrolyse der in der Haut und in den Knochen von Tieren, enthaltenen Kollagens gewonnen wird. Weichkapseln aus Gelatine ermöglichen die Verkapselung von Flüssigkeiten und Lösungen unterschiedlicher Polarität, sie bieten einen Schutz für empfindliche Hilfs- und Wirkstoffe und erlauben eine hohe Varianz möglicher Formen, Farben und Größen. Damit sind Weichkapseln den sogenannten Hartkapseln in vielerlei Hinsicht überlegen, und werden oft bevorzugt verwendet.

Trotz dieser Vorteile entstand im Verlauf der Problematik um die übertragbare spongiforme Enzephalopathien (Creutzfeld-Jakob-Disease, bovine spongioforme Enzephalopathie, Scrapie) sowie aufgrund der Diskussion um vegetarische Alternativen zu gelatinehaltigen Weichkapseln bzw. Darreichungsformen die den "koscher-" oder "hlal" - Anforderungen genügen, ein Bedarf an Weichkapseln, die ohne Verwendung von tierischen Proteinen hergestellt werden können, bzw. die aus Rohstoffen bestehen, welche nicht auf tierischen Quellen beruhen.

Als geeignete Ansgangsstoffe für die Herstellung von proteinfreien Weichkapseln wurden bereits Gele auf der Basis von Kohlehydraten beschrieben.

Als Gele werden elastische Mikrophasen im gequollenem Zustand bezeichnet. Hierbei werden die elastische Mikrophase durch Perkolation von Strukturelementen aufgebaut, die molekulare oder supermolekulare Dimension haben können und ein räumliches Netzwerk bilden. Die Gelbildung kann durch einen Spinodal- oder einen Wachstumsprozeß erfolgen. Im zweiten Fall ist der Perkolation ein Verzweigungsprozeß vorgelagert.

Nach Flory & Barett in Disc. Farad. Soc. 57, 1 (1974) werden vier Typen von Gelen unterschieden:
1. geordnete lamellare Strukturen aus Mesophasen oder silikatischen Phasen.
2. ungeordnete, kovalente, makromolekulare Netzwerke mit verzweigten und linearen Polymeren.
3. makromolekulare Netzwerke mit geordneten Vernetzungsstellen und diese verknüpfende ungeordnete Bereiche; und
4. ungeordnete Strukturen aus stark anisotropen Partikeln, Flokkulaten und Koazervaten.

Aus der Literatur sind zahlreiche Beispiele bekannt, wie Gele und Netzwerke auf der Basis von Kohlehydraten für die Verkapselung von Wirkstoffen genutzt werden können. So beschreiben Yamada, Watei & Wakao in (JP030986038) ein Verfahren zur Herstellung von Hart- und Weichkapseln bestehend aus einer Mischung von Cellulose und Stärke für die Anwendung in Lebensmitteln und pharmazeutischen Applikationen.

In der Druckschrift WO92/09274 wird der teilweise Austausch von Gelatine bei der Weichkapselherstellung durch Amylose-angereicherte Stärke vorgeschlagen. Die US 5 342 626 beschreibt die Herstellung von Filmen aus Carrageen, Gellanen und Mannanen sowie deren Verwendung zur Weichkapselherstellung. Die Nutzung von Carrageen in Konzentrationen > 5% als Geliermittel bei der Herstellung von Weichkapseln wird ebenfalls in der WO99/07347 offenbart. Die Nutzung chemisch modifizierter Stärken und Cellulosen zur Weichkapselherstellung wird in der JP93/212706 und der WO00/18835 diskutiert. Die Herstellung von Weichkapseln auf der Basis nativer (verzweigter) Kartoffelstärke wird von der EP 1 103 254 A beschrieben.

Weiterhin ist es im Fachgebiet gut bekannt, das Stärken und Mischungen aus Stärken mit weiteren Komponenten für die Herstellung von thermoplastischen Werkstoffen genutzt werden können. Diese sind beispielsweise in EP 397819, EP 542155, WO 99/02660, WO 99/02595, WO 99/02040 offengelegt. Im Unterschied zu diesen thermoplastischen Materialien weisen die in der vorliegenden Anmeldung offenbarten Gele/Netzwerke jedoch keine Fließfähigkeit oberhalb einer Glasübergangstemperatur auf. Hingegen weisen Gele bei einer Auftragung der Temperatur gegen den Logarithmus der Gibbsschen Freien Energie (log G) ein gummielastisches Plateau auf.

In der WO99/02600 der Anmelderin werden thermoplastische Mischungen auf Basis von Stärke und linearen, wasserunlöslichen Poly-α-Glucanen beschrieben. Eine Verwendung dieser Mischungen zur Herstellung von Weichkapseln bleibt unerwähnt.

Stand der Technik bei der Herstellung von Weichkapseln ist daher die Nutzung von nativen, gegebenenfalls chemisch oder physikalisch modifizierten Stärken, Cellulosen und anderen Kohlehydraten in Kombination mit weiteren geeigneten Verbindungen und gängigen Weichmachern durch verschiedene, dem gängigen Fachmann bekannte Verfahren.

Nachteilig an den bisher beschriebenen Weichkapseln auf Basis von Polysacchariden ist deren relativ geringe mechanische Festigkeit. Die bisherigen Weichkapseln auf Basis von Polysacchariden weisen Wandstärken auf, die deutlich über 100 µm und i.d.R. auch Werte von 200-300 µm überschreiten, was für einige Anwendungen von Nachteil ist, und zusätzlich einen preislichen Nachteil schafft. Weiterhin sind die verwendeten pflanzlichen Rohstoffe aufgrund ihres natürlichen Ursprungs oft sehr heterogen, was eine Produktion von einheitlichen Weichkapseln erschwert.

Aufgabe der vorliegenden Erfindung war es daher, ein Verfahren zur Herstellung von Weichkapseln zur Verfügung zu stellen, die die genannten Nachteile des Stands der Technik überwinden.

Diese Aufgabe wird durch die in den Patentansprüchen beschriebenen Ausführungsbeispiele gelöst.

Insbesondere wird diese Aufgabe gelöst durch zur Verfügungstellen eines Verfahrens zur Herstellung von proteinfreien Weichkapseln, umfassend ein Gel als Weichkapselhülle aus einem Stärkegemisch und einem Quellmittel, wobei das Stärkegemisch mindestens eine Stärkekomponente umfasst, die gegenüber nativer Stärke einen verringerten Verzweigungsgrad aufweist, und wobei das Stärkegemisch zusätzlich auch native Stärke aufweisen kann, und wobei mindestens eine der Stärkekomponenten einen Dp(N) von > 100 aufweist, dadurch gekennzeichnet dass Stärkegemisch und Quellmittel bei Temperaturen > 160°C homogenisiert werden, das erzeugte Gel in einem thermoplastischen Verarbeitungsverfahren zu einer Folie, einem Film oder einem Band verformt wird und anschließend die Weichkapsel durch das Rotary-Die-Verfahren hergestellt wird.

Diese Gemische sind insbesondere:
- Unter einem ersten bevorzugten Gesichtspunkt der vorliegenden Erfindung Gemische aus nativer Stärke und nicht-nativen, biotechnisch hergestellten, wasserunlöslichen und linearen Poly-α-Glucanen.
- Unter einem zweiten bevorzugten Gesichtspunkt der vorliegenden Erfindung Gemische aus entzweigten Stärken, wobei die Ansgangsstärke vor Entzweigung aus einer oder mehreren, unterschiedlichen Quellen stammen kann bzw. zusammen gemischt werden kann.
- Unter einem dritten bevorzugten Gesichtspunkt der vorliegenden Erfindung Gemische aus entzweigten Stärken und nativen Stärken.
- Unter einem vierten bevorzugten Gesichtspunkt der vorliegenden Erfindung Gemische aus nicht-nativen, biotechnisch hergestellten, wasserunlöslichen und linearen Poly-α-Glucanen und entzweigten Stärken.
- Unter einem fünften bevorzugten Gesichtspunkt der vorliegenden Erfindung Gemische aus nicht-nativen, biotechnisch hergestellten, wasserunlöslichen und linearen Poly-α-Glucanen, entzweigten Stärken und nativen Stärken.

Weitere bevorzugte Ausführungsbeispiele der vorliegenden Erfindung sind in den auf den Anspruch 1 zurückbezogenen Unteransprüchen beschrieben.

Unter einem weiteren bevorzugten Gesichtspunkt der vorliegenden Erfindung wird die Aufgabe gelöst durch zur Verfügung stellen Weichkapseln umfassend native Stärken mit einem hohen Amylose-Gewichtsanteil von über 0,7 (> 70 Gew.-%), wie beispielsweise Hylon® VII (National Starch and Chemical Corporation, Wilmington, DE, USA) sowie Amylogel® 3003 (Blattmann Cerestar AG, Schweiz).

Diese weisen bezogen auf Dp(N), f_{crystalline} und den Verzweigungsgrad ganz ähnliche Werte wie die erfindungsgemäßen Mischungen und sind daher voll für die Zwecke der vorliegenden Erfindung geeignet.

Weiterhin wird ein Verfahren zur Herstellung dieser Weichkapseln zur Verfügung gestellt, bei dem die oben genannten Stärkekomponenten (je nach Zusammensetzung chemisch und/oder enzymatisch entzweigten Stärken, native Stärke und/oder nicht-natives, biotechnisch hergestelltes, wasserunlösliches und lineares Poly-α-Glucan) und das Quellmittel bei Temperaturen >160°C homogenisiert werden, beispielsweise in einem Kammerkneter. Anschließend wird das erzeugte Gel in einem geeigneten thermoplastischen Verarbeitungsverfahren, vorzugsweise einer Presse oder einem Extruder, zu einer Folie, einem Film oder einem Band verformt und anschließend durch das an sich bekannte Rotary-Die-Verfahren (J.P. Stanley, Soft Gelatine Capsules; in L. Liebermann et al.: The Theory and of Practice Industrial Pharmacy; Lea & Febiger Philadelphia, 1986) zu Weichkapseln verarbeitet.

Diese Weichkapseln können beispielsweise pharmakologisch, veterinärmedizinisch, kosmetisch oder agrochemisch wirksame Substanzen oder Substanzgemische enthalten.

Weiterhin kann das Gel in einer bevorzugten Ausführungsform Geruchs- und/oder Geschmacks- und/oder die Farbe der Weichkapseln verändernde Substanzen enthalten sowie weitere Zusätze, wie sie für den jeweiligen Anwendungsfall üblich sind.

Die Herstellung erfindungsgemäß verwendbarer Gele unter Verwendung von nicht-nativem, biotechnisch hergestelltem, wasserunlöslichen und linearem Poly-α-1,4-D-Glucan wird in der PCT/EP/01/05209 der Anmelderin beschrieben.

Entzweigte Stärken können käuflich erworben werden. Ein Beispiel hierfür ist die Novelose® 330 der Firma National Starch and Chemical Corporation, Wilmington, DE, USA. Auch können entzweigte Stärken durch Einwirkung von Enzymen wie die Isoamylase oder Pullulanase auf native Stärken erzeugt werden. Diese entsprechenden Verfahren sind dem Fachmann gut bekannt. Verfahren zur enzymatischen Entzweigung von Stärken sind beispielsweise in den US 3,730,840; US 3,881,991; US 3,879,212 bzw. US 4,971,723 offenbart.

Überraschenderweise konnte von den vorstehend genannten Erfindern gezeigt werden, daß die aus einem erfindungsgemäßen Gel hergestellten Weichkapseln eine gegenüber herkömmlichen Weichkapseln stark erhöhte Festigkeit und damit verbunden zahlreiche Vorteile aufweisen.

So kann die Wandstärke der Weichkapseln um den Faktor 3 auf etwa 100 Mikrometer gegenüber gängigen polysaccharidhaltigen Produkten reduziert werden, wodurch die Weichkapseln preiswerter hergestellt werden können.

Die Dehnfähigkeit der durch die thermoplastischen Verarbeitungsverfahren erhaltenen Filme, Folien und Bänder beträgt bei dieser Wandstärke ≤ 200%, die Festigkeit bei dieser Dehnung nimmt den sehr hohen Wert von ≤ 5 MPa ein.

Als Schweißtemperatur eignen sich Bereiche von 50 - 100°C.

Darüber hinaus kann weitgehend auf Glyzerin oder andere hydrophile Polyole als Weichmacher verzichtet werden, wodurch die Hygroskopizität der Kapseln gesenkt wird. Dadurch wird die Lagerfähigkeit der Weichkapseln sowie die Sauerstoff-Barrierefunktion der Weichkapseln positiv beeinflusst.

Gegenüber gängigen gelatinehaltigen Weichkapseln bieten die offenbarten Kapseln aus biotechnisch hergestelltem, wasserunlöslichen, linearen Poly-α-1,4-D-Glucanen und Stärke neben den bereits erwähnten Vorteilen die Möglichkeit, den Wassergehalt vor der Extrusion so einzustellen, daß sie ohne weiteres Nachtrocknen der Weiterverarbeitung nach dem Rotary-Die-Verfahren zugänglich sind.

Die Erfinder konnten überraschenderweise zeigen, daß im Falle der bevorzugten Verwendung von nicht-nativem, biotechnisch hergestellten, wasserunlöslichen, linearen Poly-α-Glucanen bei der Herstellung der Gele hochgeordnete, kristalline Bereiche entstehen, die als Vemetzungsstellen für die ungeordneten Stärkemoleküle dienen. Es liegt somit nach Flory ein Typ-3 Gel vor, dessen Netzwerkdichte - bei vergleichbarem Quellungsgrad - durch den relativen Anteil an wasserunlöslichen, linearen Poly-α-Glucanen bestimmt wird. Über die Netzwerkdichte lassen sich die mechanischen Eigenschaften des Gels wie Modul, Dehnung und Spannung beim Bruch einstellen. Mithin werden in der vorliegenden Anmeldung die kristallinen Eigenschaften eingesetzten, biotechnisch hergestellten, wasserunlöslichen, linearen Poly-a-1,4-D-Glucans in vorteilhafter Art und Weise mit der guten Verarbeitbarkeit von nativer Stärke kombiniert.

Gleiches gilt unter einem weiteren bevorzugten Gesichtspunkt der vorliegenden Erfindung auch für Gele aus entzweigten Stärken wie beispielsweise der Novelose® 330 bzw. auch für Gemische aus nativer Stärke und entzweigter Stärke bzw. Gemische aus nativer Stärke, entzweigter Stärke und nicht-nativen, biotechnisch hergestellten, wasserunlöslichen, linearen Poly-α-Glucanen.

Im Rahmen der Erfindung bedeutet "nicht-nativ" nicht aus der Natur stammend. Insbesondere bedeutet dies im Falle des nicht-nativen Poly-α-Glucans das es nicht durch chemische oder enzymatische Modifikation nativer Stärke hergestellt wird.

Im Rahmen der Erfindung bedeutet "biotechnisch hergestellt" die Anwendung von biokatalytischen, auch biotransformatorischen, oder fermentativen Prozessen.

Im Rahmen dieser Erfindung bedeutet Polyglucan hergestellt durch Biokatalyse (auch: Biotransformation), daß das Polyglucan durch katalytische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird. Man spricht in diesem Zusammenhang auch von ,,in vitro Biokatalyse".

Poly-α-Glucane aus Fermentationen sind im Sprachgebrauch der Erfindung Poly-α-Glucane, die durch fermentative Prozesse unter Verwendung von in der Natur vorkommenden Organismen wie Pilzen, Algen, Bazillen, Bakterien oder Protisten oder unter Verwendung von in der Natur nicht vorkommenden Organismen, aber unter Zuhilfenahme von gentechnischen Methoden allgemeiner Definition modifizierten natürlichen Organismen, wie Pilzen, Algen, Bakterien oder Protisten gewonnen werden oder mithilfe von fermentativen Prozessen gewonnen werden können. Man spricht in diesem Zusammenhang auch von "in vivo Biokatalyse".

Beispiele für derartige Mikroorganismen sind Piichia pastoris, Trichoderma reseii, Staphylokkus carnosus, Escherichia coli oder Aspergillus niger.

Vorteilhafte Verfahren für die biotechnische Gewinnung sind z. B. in der WO 95/31553 und der WO99/67412 der Anmelderin beschrieben.

Gemäß den dort beschriebenen Verfahren wird eine Saccharoselösung mit Amylosucrase versetzt, wobei unter Spaltung der Zuckerbindung direkt Poly-α-Glucane und Fructose gebildet werden.

Weitere geeignete Enzyme sind Polysaccharidsynthasen, Stärkesynthasen, Glycoltransferasen, 1,4-D-Glucantransferasen, Glycogensynthasen oder auch Phosphorylasen.

Im Gegensatz zu Polyglucanen, die aus natürlichen Quellen wie Pflanzen isoliert werden, weisen die hierbei erhaltenen Poly-α-Glucane ein besonders homogenes Eigenschaftsprofil auf, z.B. in Bezug auf die Molekulargewichtsverteilung, sie enthalten keine oder allenfalls nur in sehr geringen Mengen unerwünschte Nebenprodukte, die aufwendig abgetrennt werden müssen oder allergene Reaktionen auslösen könnten, und lassen sich exakt spezifiziert auf einfache Weise reproduzieren.

So können bei Bedarf Polyglucane mit unterschiedlichen Eigenschaften wie Molekulargewichten etc. in definierter Weise und einfach reproduzierbar erhalten werden.

Lineare Polyglucane im Sinne der vorliegenden Erfindung sind aus Glucanen als monomeren Bausteinen derart aufgebaut, daß die einzelnen Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind lediglich die beiden Grundeinheiten, die den Anfang bzw. das Ende des Polysaccharids bilden. Diese haben nur eine Verknüpfung zu einem weiteren Monomer und bilden die Endgruppen des linearen Polyglucans.

Besitzt die Grundeinheit drei oder mehr Verknüpfungen, wird von Verzweigung gesprochen. Dabei ergibt sich aus der Anzahl der Hydroxylgruppen pro 100 Grundeinheiten, die nicht am Aufbau des linearen Polymerrückgrats beteiligt sind und die Verzweigungen ausbilden, der sogenannte Verzweigungsgrad.

Erfindungsgemäß weisen die nicht-nativen, biotechnisch hergestellten, linearen und wasserunlöslichen Poly-α-Glucane einen Verzweigungsgrad von maximal 1 % auf, d.h. sie haben maximal 1 Verzweigung auf 100 Grundeinheiten. Vorzugsweise ist der Verzweigungsgrad kleiner 0,5 % und insbesondere maximal 0,1 %.

Besonders bevorzugt sind nicht-native, biotechnisch hergestellte, wasserunlösliche, lineare Poly-α-Glucane deren Verzweigungsgrad in 6-Position kleiner 1 %, vorzugsweise maximal 0,5 % und insbesondere maximal 0,1 %, und in den anderen Positionen, z. B. in 2- bzw. 3-Position, vorzugsweise jeweils maximal 0,5 % und insbesondere maximal 0,1 % ist.

Im Falle der ebenfalls bevorzugten Verwendung von entzweigten Stärken weisen diese einen Verzweigungsgrad von maximal 0,5%, bevorzugt 0,2%, besonders bevorzugt maximal 0,03% und ganz besonders bevorzugt maximal 0,01% auf.

Für die Erfindung sind insbesondere nicht-native, biotechnisch hergestellte, wasserunlösliche, lineare Poly-α-Glucane geeignet, die keine Verzweigungen aufweisen.

In Ausnahmefällen kann der Verzweigungsgrad so minimal sein, daß er mit herkömmlichen Methoden nicht mehr nachweisbar ist.

Beispielsweise kann der Verzweigungsgrad durch NMR gemessen werden, jedoch sind dem Fachmann auch andere Methoden gut bekannt.

Weiterhin können unter einem bevorzugten Gesichtspunkt der vorliegenden Erfindung die entzweigten Stärken linear sein, wenn Sie in Mischungen mit nativen Stärke verwendet werden.

Beispiele für bevorzugte lineare Poly-α-glucane sind Poly-α-D-Glucane, wobei die Art der Verknüpfung unwesentlich ist, solange Linearität im Sinne der Erfindung vorliegt. Ein besonders bevorzugtes Beispiel ist lineares Poly-α-1,4-D-Glucan.

Für die vorliegende Erfindung beziehen sich die Präfixe "alpha" oder "D" allein auf die Verknüpfungen, die das Polymerrückgrat ausbilden und nicht auf die Verzweigungen.

Biotechnische und insbesondere biokatalytische Methoden haben den Vorteil, daß der Verzweigungsgrad kontrollierbar eingestellt werden kann und insbesondere direkt wasserunlösliche lineare Poly-α-Glucane erhalten werden können, wie z. B. die bevorzugten Poly-α-1,4- D-Glucane, die keine Verzweigungen enthalten.

Unter dem Begriff "wasserunlösliches Polyglucan" werden für die vorliegende Erfindung Verbindungen verstanden, die nach der Definition des Deutschen Arzneibuches (DAB = Deutsches Arzneibuch, Wissenschaftliche Verlagsgesellschaft mbH, Stuttgart, Govi-Verlag,' Frankfurt, Auflage, 1987) entsprechend den Klassen 4 bis 7 unter die Kategorien "wenig lösliche", "schwer lösliche", "sehr schwer lösliche" bzw. "praktisch unlösliche" Verbindungen fallen.

Erfindungsgemäß bevorzugte wasserunlösliche Poly-α-Glucane lassen sich daher der Klasse 4 des DAB zuordnen, d.h. daß eine gesättigte Lösung des Polyglucans bei Raumtemperatur und Normaldruck etwa 30 bis 100 Volumenteile Lösungsmittel, d.h. Wasser, pro Massenteil Substanz umfaßt (1g Substanz auf 30-100ml Wasser). Erfindungsgemäß mehr bevorzugte wasserunlösliche Poly-α-Glucane lassen sich der Klasse 5 des DAB zuordnen, d.h. daß eine gesättigte Lösung des Polyglucans bei Raumtemperatur und Normaldruck etwa 100 bis 1000 Volumenteile Lösungsmittel, d.h. Wasser, pro Massenteil Substanz umfaßt (1g Substanz auf 100-1000ml Wasser). Erfindungsgemäß noch mehr bevorzugte wasserunlösliche Poly-α-Glucane lassen sich der Klasse 6 des DAB zuordnen, d.h. daß eine gesättigte Lösung des Polyglucans bei Raumtemperatur und Normaldruck etwa 1000 bis 10000 Volumenteile Lösungsmittel, d.h. Wasser, pro Massenteil Substanz umfaßt (1g Substanz auf 1000-10000ml Wasser). Erfindungsgemäß am meisten bevorzugte wasserunlösliche Poly-α-Glucane lassen sich der Klasse 7 des DAB zuordnen, d.h. daß eine gesättigte Lösung des Polyglucans bei Raumtemperatur und Normaldruck etwa 10000 bis 100000 Volumenteile Lösungsmittel, d.h. Wasser, pro Massenteil Substanz umfaßt (1g Substanz auf 10000-100000ml Wasser).

"Sehr schwer löslich" entsprechend Klasse 6 kann durch folgende Versuchsbeschreibung veranschaulicht werden:
Ein Gramm des zu untersuchenden Polyglucans werden in 1 l entionisierten Wasser auf 130° C unter einem Druck von 1 bar erhitzt. Die entstehende Lösung bleibt nur kurzzeitig über wenige Minuten stabil. Beim Erkalten unter Normalbedingungen fällt die Substanz wieder aus. Nach Abkühlung auf Raumtemperatur und Abtrennung mittels Zentrifugation können unter Berücksichtigung der experimentellen Verluste mindestens 66 % der eingesetzten Menge zurückgewonnen werden.

Für die vorliegende Erfindung kann das biotechnisch erhaltene nicht-native, biotechnisch hergestellte, wasserunlösliche und lineare Poly-α-Glucane als solches eingesetzt werden. Falls erwünscht, kann es einer zusätzlichen Behandlung unterzogen werden.

So können die nicht-nativen, biotechnisch hergestellten, wasserunlöslichen, linearen Polyglucane modifiziert werden, z.B. indem die Polyglucane durch Veresterung und/oder Veretherung in einer oder mehreren nicht an der linearen Verknüpfung beteiligten Positionen chemisch modifiziert werden. Im Fall der bevorzugten 1,4 verknüpften Poly-α-Glucane kann die Modifizierung in 2-, 3- und/oder 6-Position erfolgen.

Modifikation im Sinne der Erfindung bedeutet, daß die vorhandenen Hydroxylgruppen, die nicht an der Verknüpfung beteiligt sind, chemisch verändert werden. Dies schließt eine Ringöffnung der Glucaneinheiten, wie sie z.B. bei der oxidativen Carboxylierung oder der Hydrolyse erfolgt, aus. Maßnahmen für derartige Modifizierungen sind dem Fachmann hinlänglich bekannt.

Die Poly-α-Glucane können in Form sogenannter alpha-amylaseresistenter Poly-α-Glucane eingesetzt werden wie sie am Beispiel von Poly-α-1,4-D-Glucan in den nicht Patentanmeldungen WO 00/02926 bzw. WO 01/42309 der Anmelderin beschrieben sind.

Alpha-amylaseresistente Poly-α-Glucane können durch Herstellung einer Suspension oder Dispersion aus wasserunlöslichen Poly-α-Glucanen und Wasser, Erwärmen der Suspension oder Dispersion auf eine Temperatur im Bereich von 50 bis 100 °C, Abkühlenlassen der erhaltenen kleisterartigen Mischung auf eine Temperatur im Bereich von 50 °C bis an den Gefrierpunkt, vorzugsweise 35 bis 15 °C, 27 bis 22 °C, 16 bis 0 °C oder 6 bis 2°C, über einen Zeitraum von 1 bis 72 h, vorzugsweise 1 bis 36 h und insbesondere 15 bis 30 h und Retrogradation der kleisterartigen Mischung bei einer gegenüber der Temperatur der erwärmten kleisterartigen Mischung erniedrigten Temperatur in einem Temperaturbereich von 90 bis 4 °C sowie gegebenenfalls Trocknung oder Entwässerung des erhaltenen Produktes erhaltenen werden.

Weiterhin können Alpha-amylaseresistente Poly-α-Glucane erhalten werden durch eine Inkubation unter Wasserunterschuß sowie anschließender Abkühlung und Trocknung. Dabei kann das Verfahren dadurch gekennzeichnet sein, daß man einmal oder mehrmals inkubiert, daß man das Verfahren bevorzugt bei einem Wassergehalt von 35 % durchführt und daß man die Inkubation bei einer Temperatur durchführt, die oberhalb der Glasübergangstemperatur und unterhalb der Umwandlungstemperatur liegt.

Der Polymerisationsgrad, d.h. die durchschnittliche Anzahl von Glucaneinheiten pro Molekül der erfindungsgemäß bevorzugt einsetzbaren entzweigten Stärken Dp(N) beträgt bevorzugt >10², besonders bevorzugt >10³ und ganz besonders bevorzugt >4x10³. Wird die entzweigte Stärke im Gemisch mit nativer Stärke und/oder nicht-nativem, biotechnisch hergestellten, wasserunlöslichen und linearen Poly-α-Glucan eingesetzt, kann der Dp(N) der entzweigten Stärke auch unter 100 liegen.

Der Dp(N) der erfindungsgemäß bevorzugt einsetzbaren nicht-nativen, biotechnisch hergestellten, wasserunlöslichen, linearen Poly-α-Glucane beträgt mindestens 30, bevorzugt 40 bis 300 und ganz besonders bevorzugt 50 bis 100.

Unter einem weiteren bevorzugten Gesichtspunkt der vorliegenden Erfindung weist mindestens eine Stärkekomponente des erfindungsgemäßen Stärkegemisches einen Dp(N) von >10², bevorzugt >10³ und ganz besonders bevorzugt > 4x 10³ auf.

Insgesamt sollen in dem Stärkegemisch mindestens 50 Gew.-% der mindestens einen Stärkekomponente mit Dp(N) >10² vorliegen.

Erfindungsgemäß einsetzbare entzweigte Stärken zeichnen sich ferner durch einen hohen Gewichtsanteil kristalliner Phase aus, der nach einem standardisierten Kristallisationsvorgang vorliegt. Dazu werden 5g entzeigter Stärke in 95g Wasser bei 137°C im geschlossenen System gelöst, 3 Minuten bei dieser Temperatur gehalten, die Lösung wird auf 22°C abgekühlt, und während 48 Stunden bei dieser Temperatur bei 30% Luftfeuchte gehalten. Die resultierende, im wesentliche trockenen Substanz wird in der Weitwinkel-Röntgendiffraktion untersucht. Die relative Streuintensität wird gegen den Streuwinkel zwischen 5 - 35° aufgetragen. Die Intensitätsstreuwinkel-Funktion wird nach Abzug der Fremdstreuung (Luft, Gerät) und des Beitrags der Thermischen Schwingungen der streuenden Moleküle (siehe: U.R. Trommsdorf, I. Tomka, Macromolecules 1995, 28, 8(18), 6128-6150) zwischen den Integrationsgrenzen 5 - 35°C integriert und das Integral als Iₜₒₜₐₗ bezeichnet. Der Beitrag des amorphen Halos wird von der bereinigten Intensitätsstreuwinkel-Funktion abgezogen und ebenfalls zwischen den erwähnten Grenzen integriert und dieses Integral als I_{crystalline} bezeichnet. Das Verhältnis I_{crystalline} /Iₜₒₜₐₗ wird als der Gewichtsanteil der kristallinen Phase f_{crystalline} bezeichnet. Der kristalline Anteil der untersuchten entzweigten Stärke variiert im Bereich 0,1 - 0,35. f_{crystalline} für native Stärken mit Gewichtsanteil an Amylose > 0,7 ist im Bereich < 0,12. Für native Stärken mit einem Gewichtsanteil an Amylose < 0.7 gilt f_{crystalline} < 0,1.

Der Gewichtsanteil der kristallinen Phase der entzweigten Stärke beträgt f_{crystalline} >0,1 bevorzugt >0,15, besonders bevorzugt >0,2.

Der Gewichtsanteil der kristallinen Phase der erfindungsgemäßen Stärkemischung beträgt f_{crystalline} >0,05, bevorzugt >0,1, besonders bevorzugt >0,15 und ganz besonders bevorzugt >0,2.

Betreffend der Einrichtungen, die in der vorliegenden Erfindung für die Herstellung der Kapseln eingesetzt werden, nehmen wir Bezug auf das "rotary die prozess". Die vorgeschlagene Anlage besteht aus einem Behälter (A) für die wässerigen Lösungen der modifizierten Stärke und Zuschlagstoffe, aus einer Zuleitung und Giesseinrichtung (B) für die wässerige Lösung (a), aus einem Förderband (C), auf welcher aus der Giesseinrichtung die Lösung aufgetragen wird, aus einem Förderband (C), aus einer Abdeckung (CA) für das Förderband (C), aus einer Zuführung (D) des durch Gelieren verfestigten Filmbandes, aus einem Behälter (E) mit Zuleitungskeil (F) für die in die Kapseln einzufüllende Flüssigkeit, aus einer Flüssigkeitspumpe für die Förderung des Füllgutes zwischen (E) und (F) und aus zwei gegenläufig rotierenden Formwalzen (G) mit den jeweils kapselhälftigen Aussparungen für die Ausnahme der verformten Bänder. Erhöhte Ränder an den Aussparungen sorgen für die Applikation von Druck beim Verschweissen und Ausstanzen der Kapseln. Temperatur und Förderwirkung der Teile (A) bis (G) sind kontrollierbar und regelbar.

Der Herstellungsprozeß für die Bildung von verschweissten, einteiligen Weichkapseln, insbesondere der Vorgang für die Herstellung der Filmbänder für die Weichkapselhüllen und der Abfüllvorgang stellen eine Reihe von Eigenschaftsanforderungen an die Rohstoffe. Die Filmbänder werden häufig aus durch Giessen und Abkühlen einer homogenen, molekulardispersen Lösung des Kapselhüllenmaterials erzeugt. Eine unerlässliche Anforderung an die Giesslösung ist, dass diese nach Absenken ihrer Temperatur auf einen kritischen Wert in nützlicher Zeit elastische Gel-Phasen bilden. Die Folienbänder werden am Anschluss an die Kühlzone zwischen rotierende Formwalzen geführt, gedehnt gefüllt, verschweisst und die Kapseln ausgestanzt. Die Dehnung bei der Verformung der Folienbänder betragt in Abhängigkeit der zu erzielenden Kapselform 0,85 bis 1,0. Bei der Verformung der Folienbänder entstehen Spannungen in Abhängigkeit der Dehnmodul der Bänder im Bereich 0,1 bis 10 MPa. Für die Anwendbarkeit der Folienbänder gilt die Bedingung, dass ihre Bruchdehnung und Bruchspannung jeweils größer als die oben aufgeführte Dehnung (0,85) und Spannung (0,1 - 10 MPa) ist. Um ihre Lagerfähigkeit zu verbessern werden die Kapseln getrocknet.

In der vorliegenden Erfindung wurden für die Herstellung der Kapseln aus entzweigten Stärken oder die ebenfalls bevorzugt einsetzbaren nicht-nativen, biotechnisch hergestellten, wasserunlöslichen und linearen Poly-α-Glucane folgende Parameter des Herstellungsprozesses entwickelt:
- Gewichtsanteil der verwendeten Stärken in den Lösungen (B) ist > 0,01, jedoch < 0,5, vorteilhafterweise >0,1 jedoch < 0,3.
- Die eingesetzten Stärken und Zuschlagstoffe werden bei Temperatur 50 < Tₗ < 180°C, vorteilhafterweise bei der Temperatur 50 < Tₗ < 100 °C im Wasser gelöst.
- Die Einstellung der Temperatur (Tₐ) der Giesslösung (a), der Temperatur (T_{C}) der Giessunterlage (C) und der umgebenden Luft unter der Abdeckung (CA), der Temperatur der Filmbänder (T_{f}) vor der Zuführung zu den Formwalzen, der Temperatur (Tₖ) des Zuleitungskeils (F) und der Temperatur (T_{w}) der Formwalzen ist ein wesentlicher Bestandteil des Verfahrens für die Herstellung der mit Flüssigkeit gefüllten Kapseln: 50< Tₐ < 100°C; 0< T_{c}< 30°C, 30 T_{f} < 90°C; 50 T_{w} < 100°C; 50< Tₖ < 100°C

Im Bezug auf die vorliegende Erfindung ist es notwendig, dass die eingesetzten Stärken aus heißer, wässeriger Lösung, nach deren Abkühlung elastische Gelphasen bilden mit einem Modul E > 0,1 MPa, Dehnung und Spannung beim Bruch 1,5 und > 0,1 MPa im Streckversuch (Streckgeschwindigkeit 0,1 in 10 Sekunden) bei 20 °C nach einer nützlichen Verweildauer im kalten Zustand. Die hier nicht erwähnten Zeitdauern, Lösungszusammensetzungen und Temperaturen ergeben sich aus der Beschreibung der Parameter des Verfahrens. Für die beschriebene Erfindung ist eine möglichst schnelle Bildung der elastischen Gelphase in den wässerigen Lösungen der Stärken also vorteilhaft. Die Erfinder der vorliegenden Anmeldung haben überraschenderweise gefunden, dass sich Lösungen von Stärken, die bei den oben angegebenen Prozessparametem die Anwendung des Stift-Tauch-Verfahrens gestatten, durch Angaben der chemischen Struktur und durch Parameter der Phasenstruktur charakterisieren lassen:

Der Stärkebestandteil der erfindungsgemäßen Weichkapsel kann eine beliebige Stärke oder eine Mischung aus zwei oder mehreren davon, eine oder mehrere ihrer Derivate oder Mischungen von Stärke und Stärkederivaten sein.

Geeignete Stärkebeispiele sind Stärke aus Kartoffeln, Tapioka, Maniok, Reis, Weizen oder Mais. Weitere Beispiele sind Stärken aus Maranta, Batata, Roggen, Gerste, Hirse, Hafer, Sorghum, Stärken aus Früchten wie Kastanien, Eicheln, Bohnen, Erbsen u.a. Hülsenfrüchten, Bananen, sowie Pflanzenmark zum Beispiel der Sagopalme. Sie kann entweder überwiegend Amylose oder Amylopektin enthalten, das heißt der Anteil an überwiegender Komponente ist größer 50% bezogen auf den Gesamtanteil an Amylose und Amylopektin in der Stärke. Die Stärke kann hydrothermal und/oder mechanisch vorbehandelt sein.

Neben Stärken pflanzlichen Ursprungs können auch Stärken verwendet werden, die chemisch modifiziert sind, fermentativ gewonnen wurden, rekombinanten Ursprungs sind oder durch Biotransformation beziehungsweise Biokatalyse hergestellt wurden.

Unter "chemisch modifizierten Stärken" versteht die Erfindung solche Stärken, bei denen auf chemischem Wege die Eigenschaften im Vergleich zu den natürlichen Eigenschaften verändert wurden. Dies wird im wesentlichen durch polymeranaloge Umsetzungen erreicht, bei denen Stärke mit mono-, bi- oder polyfunktionellen Reagenzien beziehungsweise Oxidationsmitteln behandelt wird. Dabei werden vorzugsweise die Hydroxygruppen der Poly-α-Glucane der Stärke durch Veretherung, Veresterung oder selektive Oxidation umgewandelt oder die Modifizierung beruht auf einer radikalisch initiierten Pfropcopolymerisation von copolymerisierbaren ungesättigten Monomeren auf das Stärkerückgrat.

Zu besonderen chemisch modifizierten Stärken gehören unter anderem Stärkeester, wie Xanthogenate, Acetate, Phosphate Sulfate, Nitrate, Stärkeether, wie zum Beispiel nichtionische, anionische oder kationische Stärkeether, oxidierte Stärken, wie etwa Dialdehydstärke, Carboxystärke, Persulfat-abgebaute Stärken und ähnliche Substanzen.

Bevorzugte chemische Modifikationen umfassen die Hydroxypropylierung, Acetylierung und Ethylierung.

"Fermentative Stärken" sind im Sprachgebrauch der Erfindung Stärken, die durch fermentative Prozesse unter Verwendung in der Natur vorkommender Organismen, wie Pilzen, Algen oder Bakterien gewonnen werden oder unter Einschaltung und Mithilfe von fermentativen Prozessen gewonnen werden können. Beispiele für Stärken aus fermentativen Prozessen umfassen neben anderen Gum Arabicum und verwandte Polysaccharide (Gellan Gum, Gum Ghatti, Gum Karaya, Gum Tragacauth), Xanthan, Emulsan, Rhamsan, Wellan, Schizophyllan, Polygalacturonate, Laminarin, Amylose, Amylopektin und Pektine.

"Stärken rekombinanten Ursprungs" oder "rekombinante Stärken" bedeutet hier Stärken, die durch fermentative Prozesse unter Verwendung in der Natur nicht vorkommender Organismen, aber unter Zuhilfenahme von gentechnischen Methoden modifizierten natürlichen Organismen, wie Pilzen, Algen oder Bakterien gewonnen werden oder unter Einschaltung und Mithilfe von fermentativen Prozessen gewonnen werden können. Beispiele für Stärken aus fermentativen, gentechnisch modifizierten Prozessen sind neben anderen Amylose, Amylopektin und weitere Poly-α-Glucane.

"Durch Biotransformation hergestellte Stärken" bedeutet im Rahmen der Erfindung, dass Stärken, Amylose, Amylopektin oder Poly-α-Glucane durch katalytische Reaktion von monomeren Grundbausteinen, im allgemeinen oligomeren Sacchariden, insbesondere Mono- und Disacchariden, hergestellt werden, indem ein Biokatalysator (auch: Enzym) unter speziellen Bedingungen verwendet wird. Beispiele für Stärken aus biokatalytischen Prozessen sind neben anderen Polyglucan und modifizierte Poly-α-Glucane, Polyfructan und modifizierte Polyfructane.

Erfindungsgemäß bedeuten die Begriffe "Derivate von Stärken" oder "Stärkederivate" ganz allgemein modifizierte Stärken, das heißt solche Stärken, bei denen zur Veränderung ihrer Eigenschaften das natürliche Amylose/Amylopektin-Verhältnis verändert wurde, einer Vorverkleisterung durchgeführt wurde, die einem partiellen hydrolytischen Abbau unterzogen wurden oder die chemisch derivatisiert wurden.

Zu besonderen Derivaten von Stärken gehören unter anderem oxidierte Stärken, zum Beispiel Dialdehydstärke oder sonstige Oxidationsprodukte mit Carboxylfunktionen, oder native ionische Stärken (zum Beispiel mit Phosphatgruppen) oder ionisch weiter modifizierte Stärken, wobei sowohl anionische als auch kationische Modifizierungen unter diesen Begriff fallen.

Neben den als Gelierungsmittel dienenden Bestandteilen enthält das erfindungsgemäße Gel einen Weichmacher oder Lösungsmittel, wobei auch hier Mischungen eingesetzt werden können, als Quellungsmittel.

Beispiele für geeignete Quellungsmittel sind Wasser, Polyalkohole wie Ethylenglykol, Glycerin, Propandiol, Erythritol, Mannitol, Sorbitol, mehrwertige Alkansäuren wie Maleinsäure, Bernsteinsäure, Adipinsäure, mehrwertige Hydroxyalkansäuren wie Milchsäure, 2-Hydroxybuttersäure, Citronensäure, Apfelsäure, Dimethylsulfoxid, Harnstoff oder weitere Lösungsmittel für Stärke.

Unter einem bevorzugten Gesichtspunkt der vorliegenden Erfindung beträgt das Verhältnis des Gewichtanteils von nicht-nativem, biotechnisch hergestellten, wasserunlöslichem, linearem Poly-α-Glucan zu Stärke in dem Gel beziehungsweise in der Weichkapsel 1% bis 50%, insbesondere 1,01% bis 30%, und das Verhältnis des Gewichtanteils an Polyglucan und Stärke zu Quellungsmittel liegt im allgemeinen im Bereich von 1% bis 60%.

Unter einem weiteren bevorzugten Gesichtspunkt der vorliegenden Erfindung beträgt das Verhältnis des Gewichtanteils entzweigter Stärke zu nativer Stärke 1% bis 50%, insbesondere 1,01% bis 30%, und das Verhältnis des Gewichtanteils an entzweigter Stärke und nativer Stärke zu Quellungsmittel liegt im allgemeinen im Bereich von 1 % bis 60%.

Im Allgemeinen liegt der Gewichtsanteil der geringer verzweigten Komponente unter dem der mehr verzweigten Komponente(n). Dieser Gewichtsanteil beträgt 1% bis 50%, bevorzugt 1,01 bis 30%. Im Gemisch aus nicht-nativem, biotechnisch hergestellten, wasserunlöslichem, linearem Poly-α-Glucan, entzweigter Stärke und nativer Stärke bedeutet dies, daß das nicht-native, biotechnisch hergestellte, wasserunlöslichem, lineare Poly-α-Glucan höchstens 50% der Gesamtheit der Kohlenhydrate ausmacht. Für die beiden anderen Komponenten gilt in diesem Fall, daß der Anteil der nativen Stärke den der entzweigten Stärke bevorzugt überwiegen soll.

Unter, einem weiteren bevorzugten Gesichtspunkt betrifft die vorliegende Erfindung aber auch Weichkapseln umfassend ausschließlich entzweigte Stärke. Je nach konkret eingesetzten Komponenten oder besonderen Anwendungsfall können diese Werte auch nach oben oder unten variieren.

Der Begriff "Weichkapsel" soll erfindungsgemäß die im Stand der Technik bekannten Produkte von kontinuierlichen und halbkontinuierlichen Herstellungsverfahren für einteilige Kapseln bedeuten. Insbesondere sollten diese Weichkapseln geeignet sein für die Umhüllung pumpbarer, im weitesten Sinne flüssiger Inhaltsstoffe im Gegensatz zu Hartkapseln, die im Allgemeinen nach Vermischen des Trägermaterials mit z.B. pulverförmigem oder hochviskosem Inhaltsstoff und Verpressen dieser Mischung hergestellt werden.

Die folgenden Beispiele erläutern die Erfindung näher.

### BEISPIEL 1.

Novelose 330®, von der Firma National Starch and Chemical Corporation, Wilmington, DE wurde eingesetzt. Dp(N), f_{crystalline} und Q_{branch} wurde für die eingesetzte Stärke Muster ermittelt und in der Tabelle 1. dargestellt. Die Einrichtung für die Herstellung der Kapseln wurde mit Formwalzen, die mit Vertiefungen für die Aufnahme der Kapselhälften in ovaler Form versehen waren ausgerüstet. Die jeweils 10 Vertiefungen an jeder Walze hatten die Länge 3 cm, die Breite 1,5 cm und die Tiefe 0,75 cm. Die Lösung (a), hergestellt aus 90 g Novelose, 10 g Glycerin und 900 g Wasser wurde bei 100° C angesetzt und für 2 Stunden bei 90° C gelagert. Die Temperaturen wurden eingestellt, der Behälter (A) der Stärkelösung (a) betrug 90° C; Tc = 3° C; Tf = 70° C; Tk = 90° C. Aus den Kapselhälften wurden Streifen geschnitten und im einfachen Zugversuch bei 20° C charakterisiert (siehe Tabelle 1.)

### BEISPIEL 2

Anstelle von Novelose wurde Hylon® VII eingesetzt und ansonsten wie in Beispiel 1 vorgegangen.

### BEISPIEL 3

Anstelle von Novelose wurde Amylogel® 3003 eingesetzt und ansonsten wie in Beispiel 1 vorgegangen.

### VERGLEICHSBEISPIEL 1

Anstatt Novelose® wurde entzweigtes Amylopectin eingesetzt und sonst wie im Beispiel 1. vorgegangen.

### VERGLEICHSBEISPIEL 2

Anstatt Novelose® wurde Kartoffelstärke Amyloplast® PE 004 von der Firma Arnylum SA Aalst, Belgium eingesetzt und sonst wie im Beispiel 1. vorgegangen.

**Tabelle 1.**

| Stärke | f_{crystaline} | Q_{branch} | Dp(N) | Spannung [MPA] | Dehnung |
|---|---|---|---|---|---|
| | | | | beim Bruch | |
| 1. Novelose® 330 | 0.30 | 5x10⁻⁴ | 2000 | 30 | 0,5 |
| Hylon® VII | 0.17 | 2x10⁻³ | 3500 | 20 | 0,2 |
| Amylogel® 3003 | 0,15 | 2x10⁻³ | 4500 | 20 | 0,2 |
| 2. Amylopectin, | 0.35 | 2x10⁻⁴ | 80 | * | * |
| entzweigt | | | | | |
| 3. Amyloplast® | 0.02 | 10⁻² | 4000 | * | * |

| | | | | | |
|---|---|---|---|---|---|
| * = keine Filme herstellbar. | | | | | |

Die Ergebnisse zeigen, daß mit entzweigter Stärke des beschriebenen Typs und mit nativer Stärke hohen Amylosegehalts >70% sehr gut entsprechende Weichkapseln hergestellt werden können. Entzweigtes Amylopektin ergibt aufgrund der zu kurzen Moleküllängen keine Filme, ebenso native Stärke nicht aufgrund des zu hohen Verzweigungsgrades und des zu geringen Anteils kritalliner Strukturen.

## Patentansprüche

1. Verfahren zur Herstellung von proteinfreien Weichkapseln, umfassend ein Gel als Weichkapselhülle aus einem Stärkegemisch und einem Quellmittel, wobei das Stärkegemisch mindestens eine Stärkekomponente umfasst, die gegenüber nativer Stärke einen verringerten Verzweigungsgrad aufweist, und wobei das Stärkegemisch zusätzlich auch native Stärke aufweisen kann, und wobei mindestens eine der Stärkekomponenten einen Dp(N) von > 100 aufweist, **dadurch gekennzeichnet dass** Stärkegemisch und Quellmittel bei Temperaturen > 160°C homogenisiert werden, das erzeugte Gel in einem thermoplastischen Verarbeitungsverfahren zu einer Folie, einem Film oder einem Band verformt wird und anschließend die Weichkapsel durch das Rotary-Die-Verfahren hergestellt wird.

2. Verfahren nach Anspruch 1, wobei das Stärkegemisch ein Gemisch aus nativer Stärke und nicht nativem, biotechnisch hergestelltem wasserunlöslichen und linearen Poly-α-Glucan ist, und wobei das Verhältnis der Gewichtsteile von Poly-α-Glucan zu nativer Stärke im Bereich von 1 Gew.-% bis 50 Gew.-% liegt.

3. Verfahren nach Anspruch 1, wobei das Stärkegemisch ein Gemisch aus entzweigten Stärken ist, und wobei die Ausgangsstärke ein homogenes Amylose/Amylopektin-Gemisch aus einer natürlichen Quelle oder ein Gemisch von Stärkekomponenten aus unterschiedlichen Quellen sein kann.

4. Verfahren nach Anspruch 1, wobei das Stärkegemisch ein Gemisch aus entzweigten Stärken und nativen Stärken ist, und wobei das Verhältnis der Gewichtsanteile von entzweigter Stärke zu nativer Stärke im Bereich von 1 Gew.-% bis 50 Gew.-% liegt.

5. Verfahren nach Anspruch 1, wobei das Stärkegemisch ein Gemisch aus nicht-nativen, biotechnisch hergestellten, wasserunlöslichen und linearen Poly-α-Glucanen und entzweigten Stärken ist.

6. Verfahren nach Anspruch 1, wobei das Stärkegemisch ein Gemisch aus nicht-nativen, biotechnisch hergestellten, wasserunlöslichen und linearen Poly-α-Glucanen, entzweigten Stärken und nativen Stärken ist, und wobei das Verhältnis der Gewichtsanteile von Poly-α-Glucan und entzweigter Stärke zu nativer Stärke im Bereich von 1 Gew.-% bis 50 Gew.-% liegt.

7. Verfahren nach einem der vorstehenden Ansprüche, **dadurch gekennzeichnet, dass** das Poly-α-Glucan Poly-α-1,4-D-Glucan ist.

8. Verfahren nach einem der vorstehenden Ansprüche, **dadurch gekennzeichnet, dass** das lineare Poly-α-Glucan biokatalytisch mit Hilfe einer Amylosucrase hergestellt wurde.

9. Verfahren nach einem der vorstehenden Ansprüche, die Weichkapsel umfassend ein Gel aus nativer Stärke mit hohem Amylosegehalt > 70 Gew.-% und einem Quellungsmittel.

10. Verfahren nach einem der vorstehenden Ansprüche, **dadurch gekennzeichnet, dass** das Gel ein Verhältnis der Gewichtsanteile aller Kohlenhydrate zu Quellmittel im Bereich von 1% bis 60% aufweist.

11. Verfahren nach einem der vorstehenden Ansprüche, **dadurch gekennzeichnet, dass** das zugrundeliegende Gel als Quellmittel mindestens einen Weichmacher enthalten kann, der ausgewählt ist aus der Gruppe bestehend aus Wasser, Ethylenglykol, Glycerin, Propandiol, Erythriol, Mannitol, Sorbitol, Maleinsäure, Bernsteinsäure, Apidinsäure, Milchsäure, 2-Hydroxybuttersäure, Citronensäure, Apfelsäure, Dimethylsulfoxid und Harnstoff.

12. Verfahren nach einem der vorstehenden Ansprüche, **dadurch gekennzeichnet, dass** das zugrundeliegende Gel essbar und/oder biologisch abbaubar ist.

13. Verfahren nach einem der vorstehenden Ansprüche, **dadurch gekennzeichnet, dass** das Gel weitere Geruchs- und/oder Geschmacks- und/oder die Farbe der Weichkapseln verändernde Substanzen enthält sowie weitere Zusätze, wie sie für den jeweiligen Anwendungsfall üblich sind.

14. Verfahren nach einem der vorstehenden Ansprüche, **dadurch gekennzeichnet, dass** die Weichkapsel pharmakologisch, veterinärmedizinisch, kosmetisch oder agrochemisch wirksame Substanzen oder Substanzgemische enthält.

15. Verfahren nach einem der vorstehenden Ansprüche, **dadurch gekennzeichnet, dass** die Weichkapseln zur Anwendung in der Nahrungs- und Genussmittelindustrie, der Medizin/Pharmazie, der Veterinärmedizin und der Agrochemie geeignet sind.

## Claims

1. Process for producing protein-free soft capsules comprising a gel as soft capsule cover of a starch mixture and a swelling agent, where the starch mixture comprises at least one starch component which has a reduced degree of branching compared with native starch, and where the starch mixture can in addition also have native starch, and where at least one of the starch components has a Dp(N) of > 100 and where starch mixture and swelling agent are homogenized at temperatures > 160°C, the gel produced is thermoformed in a processing method to give a sheet, a film or a strip, and the soft capsule is then produced by the rotary die process.

2. Process according to Claim 1 in which the starch mixture is a mixture of native starch and non-native, biotechnologically produced, water-insoluble and linear poly-α-glucan, and in which the ratio of the weight fractions of poly-α-glucan to native starch is in the range from 1% by weight to 50% by weight.

3. Process according to Claim 1 in which the starch mixture is a mixture of debranched starches, and in which the starting starch can be a homogeneous amylose/amylopectin mixture from a natural source or a mixture of starch components from different sources.

4. Process according to Claim 1 in which the starch mixture is a mixture of debranched starches and native starches, and in which the ratio of the weight fractions of debranched starch to native starch is in the range from 1% by weight to 50% by weight.

5. Process according to Claim 1 in which the starch mixture is a mixture of non-native, biotechnologically produced, water-insoluble and linear poly-α-glucans and debranched starches.

6. Process according to Claim 1 in which the starch mixture is a mixture of non-native, biotechnologically produced, water-insoluble and linear poly-α-glucans, debranched starches and native starches, and in which the ratio of the weight fractions of poly-α-glucan and debranched starches to native starch is in the range from 1% by weight to 50% by weight.

7. Process according to one of the preceding claims, **characterized in that** the poly-α-glucan is poly-α-1,4-D-glucan.

8. Process according to one of the preceding claims, **characterized in that** the linear poly-α-glucan was produced biocatalytically using an amylosucrase.

9. Process according to one of the preceding claims, the soft capsule comprising a gel of native starch having a high amylose content > 70% by weight and a swelling agent.

10. Process according to one of the preceding claims, **characterized in that** the gel has a ratio of the parts by weight of all carbohydrates to swelling agent in the range from 1% to 60%.

11. Process according to one of the preceding claims, **characterized in that** the underlying gel can comprise as swelling agent at least one plasticizer which is selected from the group consisting of water, ethylene glycol, glycerol, propanediol, erythritol, mannitol, sorbitol, maleic acid, succinic acid, adipic acid, lactic acid, 2-hydroxybutyric acid, citric acid, malic acid, dimethyl sulfoxide and urea.

12. Process according to one of the preceding claims, **characterized in that** the underlying gel is edible and/or biodegradable.

13. Process according to one of the preceding claims, **characterized in that** the gel comprises further substances modifying odour and/or taste and/or colour of the soft capsules, and other additives as are customary for the respective application.

14. Process according to one of the preceding claims, **characterized in that** the soft capsule comprises substances or mixtures of substances which are pharmacologically active, active in veterinary medicine, cosmetically active or agrochemically active.

15. Process according to one of the preceding claims, **characterized in that** the soft capsules are suitable for use in the food and drink industry, medicine/pharmacy, veterinary medicine and agrochemistry.

## Revendications

1. Procédé pour obtenir des capsules souples sans protéines, qui contiennent un gel comme enveloppe de capsules souples d'un mélange d'amidon et un agent de gonflement, où le mélange d'amidon contient au moins une composante d'amidon, qui présente un degré de ramification plus réduit à l'égard de l'amidon natif, et où le mélange d'amidon peut présenter de manière supplémentaire aussi l'amidon natif, et où au moins l'une d'entre les composantes d'amidon présente un Dp(N) de > 100, **caractérisé en ce que** le mélange d'amidon et l'agent de gonflement sont homogénéisés aux températures > 160°C, le gel produit sera formé par un procédé de traitement thermoplastique dans une feuille, un film ou une bande et puis on obtient la capsule souple par le procédé Rotary-Die.

2. Procédé selon la revendication 1, où le mélange d'amidon est un mélange d'amidon natif et de poly-α-glucan non-natif, linéaire et insoluble dans l'eau, obtenu par un procédé biotechnique, et où le rapport de poids des parts de poly-α-glucan à l'amidon natif se trouve dans le domaine de 1% en poids à 50% en poids.

3. Procédé selon la revendication 1, où le mélange d'amidon est un mélange d'amidons non-ramifiés et où l'amidon initial peut être un mélange homogène d'amylose/amylopéctine d'une source naturelle ou un mélange de composantes d'amidon de différentes sources.

4. Procédé selon la revendication 1, où le mélange d'amidon est un mélange d'amidons non-ramifiés et d'amidons natifs, où le rapport de poids des parts d'amidon non-ramifié à l'amidon natif se trouve dans le domaine de 1% en poids à 50% en poids.

5. Procédé selon la revendication 1, où le mélange d'amidon est un mélange de poly-α-glucans non-natifs, linéaires et insolubles dans l'eau, obtenus par un procédé biotechnique et d'amidons non-ramifiés.

6. Procédé selon la revendication 1, où le mélange d'amidon est un mélange de poly-α-glucans non-natifs, linéaires et insolubles dans l'eau, obtenus par un procédé biotechnique, d'amidons non-ramifiés et d'amidons natifs, et où le rapport de poids des parts de poly-α-glucan et d'amidon non-ramifié à l'amidon natif se trouve dans le domaine de 1% en poids à 50% en poids.

7. Procédé selon l'une des revendications de ci-dessus, **caractérisé en ce que** le poly-α-glucan est le poly-α-1,4-D-glucan.

8. Procédé selon l'une des revendications de ci-dessus, **caractérisé en ce que** le poly-α-glucan linéaire a été obtenu par un procédé biocatalytique à l'aide d'une amylosucrase.

9. Procédé selon l'une des revendications de ci-dessus, la capsule souple contenant un gel d'amidon natif avec une teneur élevée d'amylose > 70% en poids et un agent de gonflement.

10. Procédé selon l'une des revendications de ci-dessus, **caractérisé en ce que** le gel présente un rapport de poids des parts d'entre tous les hydrates de carbone et l'agent de gonflement dans le domaine de 1% à 60%.

11. Procédé selon l'une des revendications de ci-dessus, **caractérisé en ce que** le gel sur lequel est basé peut contenir comme agent de gonflement, au moins un plastifiant, qui est choisi du groupement formé de l'eau, de l'éthylèneglycol, de glycérine, de propanediol, de l'érythriol, de mannitol, de sorbitol, de l'acide maléique, de l'acide succinique, de l'acide adipique, de l'acide lactique, de l'acide 2-hydroxybutirique, de l'acide citrique, de l'acide malique, de diméthylsulfoxyde et d'urée.

12. Procédé selon l'une des revendications de ci-dessus, **caractérisé en ce que** le gel sur lequel est basé est comestible et/ou biodégradable.

13. Procédé selon l'une des revendications de ci-dessus, **caractérisé en ce que** le gel sur lequel est basé contient aussi d'autres substances pour odeur et/ou pour goût et/ou qui modifient la couleur des capsules souples aussi bien que d'autres suppléments, tels qu'ils sont usuels pour chaque cas d'utilisation.

14. Procédé selon l'une des revendications de ci-dessus, **caractérisé en ce que** la capsule souple contient des substances ou des mélanges de substances actives pharmacologiques, médicinal-vétérinaires, cosmétiques ou agrochimiques.

15. Procédé selon l'une des revendications de ci-dessus, **caractérisé en ce que** les capsules souples sont adéquates pour utilisation dans l'industrie alimentaire et de délicatesses, dans la médecine/pharmacie, dans la médecine vétérinaire et dans l'agrochimie.
